# EUROPEAN PATENT APPLICATION

(11) **EP 1 992 322 A1**
(43) Date of publication of application: **19.11.2008**
(21) Application number: 07011232.1
(22) Date of filing: 08.06.2007
(51) Int. Cl.: A61K 8/02, A61K 8/55, A61K 8/97, A61Q 19/08

(54) **Composition for percutaneous application**

(30) Priority: 11.05.2007 EP 07009487
(71) Applicant: Dr. Scheller Cosmetics AG, 73054 Eislingen (DE)
(72) Inventor: The designation of the inventor has not yet been filed
(74) Representative: Gesthuysen, von Rohr & Eggert

(57) **Abstract**

Composition for percutaneous application, particularly for use on ageing and/or stressed skin, which contains
a) Water;
b) At least two percutaneously-applicable active substances and
c) At least one substance which with water forms a lamellar structure, and which contains at least one functional group of the general formula I

-CH₂-N⁺-(CH₃)₃, (Formula I),

whereby the composition
b-1) contains at least one plant extract containing at least one phytohormone, and
b-2) at least one anti-ageing agent other than b-1.

Preferred areas of application of the composition include its use to produce a cosmetic composition and its use to produce a pharmaceutical composition to change the hormone level in a human.

## Description

The present invention concerns a composition for percutaneous application with the characteristics of the preamble to patent claim 1 and particularly a composition for percutaneous application to ageing and/or stressed skin.

Compositions for percutaneous application have long been known and include both cosmetic compositions, the active substances of which penetrate the skin, at least in part, and pharmaceutical compositions, the active substances of which permeate the skin, at least in part.

Cosmetic compositions, particularly cosmetic compositions used for the care and/or treatment of the skin, and preferably for ageing and/or stressed skin, usually contain water and substances which can form gels or emulsions with water. Such an aqueous emulsion, to which it may be possible to add a lipophilic additive, represents the simplest case of a cosmetic composition.

Furthermore, such a cosmetic composition may contain further cosmetic active substances, thickening agents, gel formers, colourings, stabilisers, ageing inhibitors, scents and/or pH regulators.

WO 01/62222 describes a cosmetic composition, particularly for application to ageing and/or stressed skin, the composition comprising, in addition to water, at least one substance that forms lamellar structures with water. The composition further comprises
a) at least one compound that contains a functional group of the general formula I

   - CH₂ - N⁺ - (CH₃)₃, (Formula I),
b) and/or at least one metabolite of said compound
c) and/or S-adenosylmethionine.

The composition contains at least one substance as a cosmetic active substance which cleans and cares for the skin, and maintains a healthy skin condition, secures external protection of the skin from harmful environmental pollution, climatic and actinic effects, particularly excessive solar and UV radiation, protection of the skin from detergents and cleaning agents and other environmental effects, particularly dust and emissions. In particular, this means unsaturated fats which have an elasticising effect, liquid fatty acid esters and hydrocarbons with short-chain branches, which have a spreading effect, screening and protective fats, which include, in particular, oils, liquid fatty alcohols, silicon oils, solid fatty acid esters and/or fatty alcohols, vitamins, oligoproteins, collagen hydrolysate and UV filter substances.

Pharmaceutical compositions which may be applied percutaneously usually contain water, an active substance which may be applied percutaneously and at least one carrier substance for the active substance. This carrier substance is used to transport the active substance through the structures of the skin, so that a pharmaceutical composition applied in this way, avoiding the gastrointestinal tract and a corresponding injection, carries the active substance to where its pharmaceutical effect is to be deployed locally and/or systemically. In order to accelerate the transport of the active substance through the structures of the skin whilst preventing undesirable breakdown of the active substance, the active substance is frequently encapsulated in the carrier substance present as a vesicle or attached to it, such spherical vesicles usually being called liposomes, nanosomes and/or cerasomes.

Moreover, such a pharmaceutical composition may contain further ingredients, e.g. thickening agents, gel formers, stabilisers, colourings, ageing inhibitors, scents and/or pH regulators.

DE 198 57 490 proposes a formulation for protecting skin against external agents acting upon the skin and damage of endogenous origin which contains:
1) Saturated phosphatidyl choline 0.10-10% by weight;
2) Unsaturated phosphatidyl choline 0.01-1.00% by weight;
3) Medium-chain triglycerides 1.00-50% by weight;
4) Salts 0.01-10.00% by weight;
5) Moisture retainers 1.00-20.00% by weight
6) Dermatological or cosmetic active agents 0.01-20.00% by weight, and:
7) Water 20.00-95.00% by weight,
whereby the unsaturated/saturated phosphatidyl choline ratio is 0.001-0.2

Plant and yeast extracts are identified as dermatological or cosmetic active substances.

WO 02/089770 describes a pharmaceutical composition which contains water, at least one topically applicable active substance and at least one carrier substance for the active substance, the carrier substance being a substance which, with water, forms a lamellar double membrane structure, whereby this lamellar double membrane structure is provided in the composition and whereby the composition also comprises at least one compound which has at least one functional group of general formula I

- CH₂ - N⁺ - (CH₃)₃, (Formula I),

and/or comprises at least one metabolite of this compound and/or S-adenosylmethionine, whereby at least one N-acylethanolamine is provided in the composition.

The composition contains at least one topically-applicable active substance. Possible examples quoted include cortisone, cortisone derivatives, particularly progesterone, testosterone, aldosterone, hyprocortisone and cortisol.

It is also known that certain plant substances, the so-called phytohormones, have a corresponding affinity to the corresponding human hormone receptors, due to some structures being similar.

In consideration of this state of the art, the present invention is based upon the problem of providing a composition for percutaneous application which protects the human skin, particularly women's skin, as effectively as possible from ageing, and restores ageing, diseases and/or stressed skin as quickly and completely as possible.

This problem is solved according to the invention by a composition with the characteristics of claim 1. Particularly practical embodiments of the invention are described in the sub-claims.

By providing a composition which contains
a) Water;
b) At least two percutaneously-applicable active substances and
c) At least one substance which forms a lamellar structure with water and which contains at least one functional group of the general formula I

   - CH₂ - N⁺ - (CH₃)₃, (Formula I),

   whereby the composition
   b-1) contains at least one plant extract containing at least one phytohormone, and
   b-2) at least one anti-ageing agent other than b-1).
it is succeeded to render accessible a composition for percutaneous application in a way which cannot easily be foreseen, particularly for use on ageing and/or stressed skin, protecting human skin, particularly women's skin, as effectively as possible from ageing, and restores ageing, diseased and/or stressed skin comparatively quickly and completely. Surprisingly, the present invention facilitates specific correction of hormonal skin ageing at any age. Phytohormones compensate for hormonal changes at least in part and counteract the cause of skin ageing, so that skin treated with the composition according to the invention is or becomes elastic and smooth, and skin already irritated and/or stressed regains its original appearance.

Moreover, it may be observed that aged skin is returned to a taut, elastic condition, so that unwanted wrinkles in particular are smoothed out. Even after only a few applications of the composition according to the invention, dry or roughened skin regains its natural, elastic appearance.

The present invention achieves thoroughly efficient protection of the skin from ageing. The compositions according to the invention pose no health risks whatsoever and are free of side effects.

Furthermore, the compositions according to the invention are distinguished by extremely high storage stability.

In principle, the water content of the composition according to the invention is a matter of discretion and is preferably in the range of 1% - 99% by weight, particularly in the range of 5% - 90% by weight, expressed as a percentage of the total weight of the composition.

The term "water" used in this application means any aqueous system, i.e. particularly sterilised water, deionised water, distilled water, aqueous solutions and/or aqueous buffer systems.

The composition according to the invention includes at least two percutaneously-applicable active substances,
b-1) at least one plant extract including at least one, preferably at least two, phytohormone(s), and
b)-2) at least one anti-ageing agent which differs from b-1).

Secondary plant constituents are preferably used as active substances, which the plants use for an absolutely specific function, e.g. as antibodies, colouring or growth regulators, and which exercise a specific biological function in the human body. A distinction must be made between them and primary constituents, e.g. fats, oils and proteins, which are predominantly active in energy metabolism and anabolism

Plant extracts means the active substance extracts obtained by extraction from plants and preferably partial or total evaporation of the extraction solution. Hydrophilic solvents are particularly preferred as extraction agents, especially water and alcohol with 1 to 6 carbon atoms, particularly water and ethanol, lipophilic solvents, particularly fats, oils, aliphatic hydrocarbons with 2 to 10 carbon atoms, particularly n-hexane and cyclohexane, and supercritical solvents, particularly supercritical carbon dioxide.

The plant extract according to the invention contains at least one phytohormone, i.e. at least one plant constituent, which has a corresponding affinity to the corresponding human hormone receptors, due to structures partially similar to human hormones. This particularly includes those substances which can trigger effects similar to those of hormones in the human organism, particularly in women. For the purposes of the present invention, those phytohormones are particularly preferred which can influence the oestrogen, progesterone and/or testosterone levels, but particularly the oestrogen level.

Suitable phytohormones include
- Stilbene compounds, particularly resveratrol
- Lignan compounds, particularly enterodiol, and
- lsoflavone, particularly genistein, daizein, glycitein, formononetin, biochanin A, equol and glycosides of these compounds.

Particularly preferred b-1) plant extracts may be obtained from the plants Cimicifuga racemosa, Trifolium pratense, Angelica sinensis, Panax ginseng, Piper methysticum, Glycine max, Dioscorea spec., Pelvetia spec., Humulus lupulus, Glycyrrhiza glabra, Urtica dioica, Sabal serrulata, Serenoa repens, Secale cereale, Avena sativa, Cucurbita pepo and Hypoxis rooperi.

The phytohormone content in the plant extract is advantageously less than 80% by weight, preferably less than 70% by weight, particularly preferably less than 60% by weight, particularly less than 50% by weight in terms of the dry weight of the extract following drying to a constant weight at 105°C for at least three hours. The phytohormone content is also preferably less than 20% by weight under these conditions. If the plant extract contains several phytohormones, the figures refer to the proportion of all the phytohormones in the extract.

The proportion of the plant extract in the composition according to the invention is preferably selected so that, in percutaneous application of a normal quantity of the composition according to the invention, the hormone level affected by the plant extract in the tissue treated is at least partially balanced out.

Within the scope of a particularly practical embodiment of the present invention, the composition contains at least two plant extracts, preferably influencing two different hormone levels, particularly those of oestrogen and progesterone, the ratio of the plant extracts preferably being selected according to the need following adjustment of the respective hormone level.

The following plant extract mixtures have proved particularly suitable for the purposes of the present invention:
Trifolium pratense:Dioscorea spec. in a ratio of 1:100 to 100: 1, preferred in a ratio of 1:20 to 20:1, particularly for percutaneous application in women aged 45 to 60 years.
Glycine max: Pelvetia spec. in a ratio of 1:100 to 100:1, preferred in a ratio of 1:20 to 20:1, particularly preferred in a ratio of 1:5 to 5:1, particularly for percutaneous application in women aged over 60 years.
The figures refer to the respective dry weights, which can be determined by drying to constant weight at 105°C for at least three hours.

"Anti-ageing agents" (ageing inhibitors) means substances which delay the biological ageing of the human skin, maintain its quality at a high level for as long as possible and also extend the overall life of the skin. This expressly includes substances which delay the biological ageing of the skin, which is attributable to a decrease in the speed of cell regeneration, a loss of the capability to retain moisture and/or to increasing collagen loss in lower layers of the skin.

For the purposes of the present invention, particularly effective anti-ageing agents include:
- Provitamins, particularly carotinoids, particularly preferably β-carotin, lycopin, ascorbic acid (vitamin C), vitamins E and D;
- Phenolic compounds, particularly flavonoids, particularly preferably quercetin and xanthohumol, tannins, particularly emblicanin A, emblicanin B, pedunculagin and penigluconin, gallotannins, particularly preferably epicatechin and ellagic acid, curcuminoids, proanthocyanidins, particularly preferably pycnogenol;
- Organosulphur compounds, particularly cysteine sulphoxides, glucosinolates and mustard oils (isothiocyanates);
- Terpenoids, particularly monoterpene, particularly preferably limes, perillyl alcohol, triterpenes, particularly preferably urolic acid.

The above-mentioned phytohormones do not form part of the group of anti-ageing agents.

Particularly preferred anti-ageing agents include Punica granatum extract, vitamin E, alpha lipoic acid, Phyllanthus emblica extract and Vitis vinifera extract, particularly Punica granatum extract, Phyllanthus emblica extract and Vitis vinifera extract.

Particularly favourable results using anti-ageing agents and by the use of resveratrol as an anti-ageing agent, may be obtained from the following fruits: Apples (Malus domestica), pears (Pyrus communis), quinces (Cydonia oblonga), morello cherries, (Cerasus vulgaris), sweet cherries (Cerasus avium) plums (Prunus domestica), apricots (Armeniaca vulgaris), peaches (Persica vulgaris), gooseberries (Ribes uva-crispa), blackcurrants (Ribes rubrum), raspberries (Rubus idaeus), blackberries (Rubus fruticosus), strawberries (Fragaria spec.), rosehips (Rosa spec.), pineapples (Ananas comosus), figs (Ficus carica), bilberries (Vaccinium myrtillus and other species of vaccinium), cranberries (Vaccinium vitis-idaea), elderberries (Sambucus nigra), cornelian cherries (Cornus mas), sallow thorn (Hippophae rhamnoides), sloes (Prunus spinosa), Oregon grapes (Mahonia aquifolium), mangoes (Mangifera indica), Barbados cherries (Malpighia glabra), melons (Cucumis melo), water melons (Citrullus lanatus), pomegranates (Punica granatum), passion fruit (Passiflora edulis), durian fruit (Durio zibethinus), akees (Blighia sapida), lychees (Litchi chinensis), rambutans (Nephelium lappaceum), mangosteens (Garcinia mangostana), cashew apples (Anacardium occidentale), tamarinds (Tamarindus indica), grapes (Vitis vinifera), bananas (Musa spec.), dates (Phoenix dactylifera), papayas (Carica papaya), guavas (Psidium guajava), Japanese persimmon (Diospyrus kaki), kiwis (Actinidia chinensis), Cape gooseberries (Physalis), prickly pears (Opuntia ficus-indica), star fruit (Averrhoa carambola), oranges (Citrus sinensis), mandarins (Citrus deliciosa), grapefruit (Citrus paradisi), shaddock (Citrus maxima), lemons (Citrus limon), citrons (Citrus medica), key limes (Citrus aurantiifolia), bitter oranges (Citrus aurantium) and kumquats (Fortunella margarita).

Moreover, the use of plant extracts in this connection has proved particularly effective. The anti-ageing agent content in the plant extract is then advantageously less than 55% by weight, practically less than 20% by weight and particularly preferably less than 20% by weight in terms of the dry weight of the extract following drying to constant weight at 105°C for at least three hours. Should the plant extract contain several anti-ageing agents, the figures refer to the proportion of all the anti-ageing agents in the extract.

The concentration of the active substances listed above present in the composition according to the invention is preferably between 0.05% and 20% by weight, particularly preferably between 0.1% and 4% by weight, in terms of the weight of the composition.

The proportion of phytohormones is preferably in the range from 0.001% by weight to 5% by weight, preferably in the range from 0.01% by weight to 2% by weight and particularly preferably in the range from 0.05% by weight to 1.5% by weight in terms of the total weight of the composition.

The proportion of the anti-ageing agents is preferably in the range from 0.001% by weight to 5% by weight, preferably in the range from 0.01% by weight to 2% by weight, preferably in the range from 0.05% by weight to 1.5% by weight, in terms of the total weight of the composition.

The ratio of phytohormones to anti-ageing agents is preferably selected in the range from 10:1 to 1:10, particularly in the range from 1:5 to 5:1 in terms of the proportion of these constituents by weight.

The composition according to the invention may contain further active substances, in addition to those identified above. These particularly include cosmetic active substances, i.e. especially substances which entail cleansing and care of the skin and maintenance of a healthy skin condition, external protection of the skin from harmful environmental, climatic and actinic influences, in particular, for example, excessive solar and UV radiation, detergents and cleaning agents and other environmental pollution, particularly dust and emissions. Particularly preferred cosmetic active substances include unsaturated fats which have an elasticising effect, liquid fatty acid esters and hydrocarbons with short-chain branches, which have a spreading effect, screening and protective fats, which include, in particular, oils, liquid fatty alcohols, silicon oils, solid fatty acid esters and/or fatty alcohols, whereby an oil and/or an oil constituent, particularly the non-saponifiable constituents of a vegetable oil, preferably such as avocado oil, olive oil and/or at least one native oil is preferably contained in the inventive cosmetic composition as a cosmetic active substance. Furthermore, vitamins, olegoproteins, collagen hydrolysate and known and established UV filter substances must be identified as cosmetic active substances.

The concentration of the cosmetic active substances listed above favourably lies between 0.01% by weight and 55% by weight and particularly between 5% and 30% by weight, in terms of the total weight of the composition.

In another embodiment of the composition according to the invention, it has, in particular, at least one further active substance, in addition to or instead of those listed above, which increase skin moisture in percutaneous application. In general, N-acyl-alkanolamines, preferably lactamide MEA, oleamide MEA and/or acetamide MEA, and particularly N-acyl ethanolamines, e.g. especially N-acetyl-phosphatidylethanolamine, N-acetyl-ethanolamine, N-linolenoyl ethanolamine, N-acyl ethanolamin and/or N-alcyl-2-hydroxy-propylamine, the two last-named compounds then containing alcyl residues in the form of fatty acids from coconut oil and/or palm oil.

The concentration of these active substances which increase skin moisture in the composition according to the invention is preferably between 0.5% and 20%, in terms of the total weight of the composition respectively.

Within the scope of a particularly preferred embodiment of the present invention, the composition contains a glycosaminoglycane, possibly in the form of a physiologically unobjectionable salt, particularly hyaluronic acid, to further enhance the anti-wrinkle effect of the composition.

The proportion of the hyaluronic acid (salts) is preferably in the range from 0.001% by weight to 1.0% by weight, particularly in the range from 0.05% by weight to 0.5% by weight in terms of the total weight of the composition.

As well as water and the at least two percutaneously-applicable active substances, the composition according to the invention contains at least one substance c), which contains at least one functional group of general formula I

- CH₂ - N⁺ - (CH₃)₃, (Formula I).

In the composition according to the invention, the substance c) forms lamellar structures with water, particularly a lamellar double membrane structure, which has a layered structure. One respective upper layer of the substance c) is preferably oriented towards a lower layer of the substance c) and the orientation of the individual substance layers takes place favourably so that the hydrophilic residues of substance c) are respectively outwards and thus towards the aqueous phase surrounding these substance layers, whilst the lipophilic residues of substance c) are aligned with each other inwardly, which is generally deemed a double membrane. If at least two double membrane layers are then aligned above each other in sandwich form, depending upon the concentration of the substance c), layered double membranes are formed, said layered double membranes containing, for example, two to ten double membranes separated by at least one layer of water, aligned with each other in parallel, predominantly planar in extension. Both the double membrane described above and the layered double membrane may be present in the composition according to the invention as a lamellar structure and are generically referred to as a lamellar double membrane structure in this application. For the sake of clarity, it should be noted that other structures, e.g. the bullous and spherical simple membrane structures (liposomes, transfersomes) described above may be present in the composition according to the invention, in addition to the lamellar membrane structures. However, such bullous and spherical bullous membrane structures are not wanted in the composition according to the invention and should be avoided if possible.

The structure of the composition has already been clearly explained in WO 02/089770. Figs. 1 and 2 of this application show the structure of a double membrane structure and of double membranes arranged in sandwich form. Explicit reference is thus made to the disclosure of this specification, particularly to page 22, line 20 to page 23, line 19, and to drawings 1 and 2.

Application of the composition according to the invention is highly effective. The lamellar membrane structures formed with water from substance c) have similar physical properties to the lipids of the skin permeability barrier and particularly the lipid areas of the stratum corneum. The composition according to the invention cannot have a negative effect upon the permeability barrier, due to its similar physical properties and identical or similar arrangement to it, which is firstly expressed by simplified permeation and penetration by the inventive compound in and through the skin barriers and secondly by the lack of a negative effect on and/or damage to these skin barriers.

This itself has the consequence that the substance c) present in a certain (lamellar membrane) structure in the composition according to the invention transports the active substance into or through the corresponding skin barriers particularly quickly and evenly, without causing damage to or irritation of the skin, and particularly while avoiding the transdermal water loss occurring in established agents, so that, in particular, side effects also caused by percutaneous application of the composition according to the invention are considerably minimised and, in particular, do not even arise.

The concentration of the active substance in the inventive solution can be reduced by comparison with the established liposomal composition, due to the greater effectiveness of the composition according to the invention described above.

The greater effectiveness of the composition according to the invention also means that the composition according to the invention can be used particularly well for systemic treatment, as permeation of the composition according to the invention into those areas of the epidermis below the stratum corneum is facilitated by the previously-specified special lamellar membrane structure of the substance c).

In addition, the composition according to the invention displays an increased absorption capacity for both hydrophilic and lipophilic active substances with increased storage stability, compared to known compositions. This is associated with the lamellar membranes being aligned with each other in such a way that layers of water are formed between adjacent double membranes which can thus absorb high concentrations of water-soluble active substances, whilst the lipophilic active substances themselves are located directly inside the double membranes.

The term "percutaneous application" covers all forms of application of the composition according to the invention in which it is applied to the skin, specifically including the skin of the face, hands, thighs and cleavage are included.

Fundamentally, it must be stated that the composition according to the invention in the form of substance c) forming lamellar membrane structures of the type described above with water contains those substances which display a hydrophilic and simultaneously hydrophobic molecular residue.

In this respect, monoglycerides, diglycerides, particularly distilled medium-chain monoglycerides, sphingolipids, lecithin, phospholipids, fatty alcohols, fatty acids, soaps, mono- and/or diesters of fatty acids, sucrose, glucose and/or their derivatives, glucosidal, furanosidal and/or pyranosidal condensation products of fatty alcohols with glucose and/or sucrose and their polymer derivatives, mono- and/or diesters of glucosides with fatty acid derivatives, steroids, mono- and/or diesters of fatty acids and steroids and/or glycol derivatives from steroids should be expressly mentioned, the fatty acids preferably having a C₈-C₂₄ saturated, linear carbon chain.

However, it is particularly suitable if substance c) in the composition according to the invention, which is in a position to form the aforementioned lamellar membrane structures with water, contains at least one hydrated lecithin and/or a hydrated phospholipid, and particularly a hydrated phosphatidylcholine. In this case it was possible to establish that such hydrated phospholipids, and particularly the hydrated phosphatidylcholine, firstly form lamellar membrane structures to a high degree and secondly that these lamellar membrane structures are outstandingly suited to migration into the intercellular lipids of the stratum corneum and/or even to penetrate them without damage occurring to the lipid layer.

However, such a hydrated lecithin or such a hydrated phospholipid is preferably provided in the composition according to the invention, in which all the acyl residues are exclusively or predominantly saturated, so that, in particular, only unsaturated acyl residues are present in a concentration of less than 10% by weight and preferably less than 5% by weight, and very preferably less than 1.5% by weight, in the hydrated lecithin, the hydrated phospholipid and/or particularly in the phosphatidylcholine.

For the sake of clarity it should be stated that the term "phospholipid" includes, of course, not only a single phospholipid, but also a mixture of phospholipids, whereby the phospholipids or mixture of phospholipids may be of natural or synthetic origin. It is also obvious that the phospholipid is not hydrated in the above sense, but that a synthetic phospholipid, in which most or all of the acyl residues in the above sense are saturated, is used instead of this hydrated phospholipid.

The advantages of such further developments of the composition according to the invention described above which contain substance c) in the form of a hydrated phospholipid which itself contains at least 60% hydrated phosphatidylcholine by weight, preferably between 70% and 99% by weight, particularly more than 90% by weight, in terms of the total weight of phospholipid in the composition, have the above-mentioned advantages to an increased extent.

In respect of the concentration of the at least one substance c) contained in the composition according to the invention which can form lamellar membrane structures with water, it may generally be stated that this concentration depends upon the transport and storage capacity of the lamellar membrane structures to be formed correspondingly for the active substances and for the additional constituents of the composition according to the invention. In particular, this at least one substance c) is present in the composition according to the invention in a concentration of between 0.5% by weight and 30% by weight, preferably in a concentration between 1% by weight and 15% by weight, in terms of the total weight of the composition.

Particularly when the hydrated phospholipid, hydrated phosphatidylcholine or hydrated lecithin, or a corresponding synthetically produced phospholipid with correspondingly saturated acyl residues described above has a phase transition temperature over 30°C and below 70°C, an embodiment of the composition according to the invention displaying the desired lamellar membrane structures extensively described above can be produced particularly simply using such a substance c). The phase transition temperature is specified so that it expresses the temperature at which the crystalline system changes to a liquid system, whereby this temperature does not identify a specific individual temperature but a range of temperatures in many cases. For example, the phase transition temperature for the particularly preferred phosphatidylcholine, which is isolated from soya beans and which has a phosphatidylcholine concentration of 93 ± 3% by weight, and the acyl residues of which consist of 85% by weight of stearic acid and 14% by weight of palmitic acid, is between 54°C and 58°C, particularly 56°C.

If plant products are used as substance c), preferably isolates with a compound c) concentration greater than 80% by weight in terms of the total weight of the plant extract are used.

In respect of the compound c) contained in the composition according to the invention, it must be stated that the compound, which contains at least one functional group of the above-mentioned general formula 1, be such a compound which is naturally present in aerobic cells, particularly in the cell membranes. However, at this point it should be expressly emphasised that the composition according to the invention should not, as a compound, contain any chemical constituents which are technically known as quaternary ammonium compounds and which represent synthetic surface-active substances.

It is particularly advantageous, if the composition according to the invention contains betaine, acetylcholine, choline, glycerophosphocholine, phosphatidylcholine, lysophosphatidylcholine, carnitine, acylcarnitine or sphingomyeline, either alone or mixed with each other, and/or their derivatives as compound c).

The concentration of the compound c) with the functional group shown in formula I contained in the composition according to the invention depends upon the respective application of the composition according to the invention.

It is particularly preferable if the compound c) is present in a concentration of between 0.0001% by weight and 10% by weight, preferably between 0.1% by weight and 9% by weight, in terms of the total weight of the composition in each case.

The composition according to the invention also preferably contains between 15% by weight and 99% by weight, preferably between 30% by weight and 95% by weight, of lamellar structures, particularly lamellar double membranes, in terms of the total weight of the substance c) in the composition.

The composition according to the invention preferably has lamellar structures in which each individual double membrane is between 4 nm and 20 nm thick, preferably between 4 nm and 8 nm thick.

A particularly advantageous further development of the above composition according to the invention, has a fatty acid, a fatty acid salt and/or a mixture of betaine and at least one fatty acid salt and/or a mixture of betaine with at least one fatty acid salt as compound c).

The salt selected as a fatty acid salt is preferably one in which the original fatty acid is preferably linear or a mixture of fatty acids, and has between 12 and 24 carbon atoms, whilst any fatty acid used preferably also has between 12 and 24 carbon atoms.

Particularly suitable fatty acid salts of betaine are betaine laurate, betaine myristate, betaine palmitate, betaine stearate, betaine oleate, betaine linolate, alone and in mixtures thereof. In this case it was surprisingly established that particularly these previously specifically-mentioned fatty acids of betaine give the composition according to the invention particularly high cosmetic effectiveness, despite their relatively poor solubility in water.

The composition according to the invention may contain further known additives, such as preservatives, antioxidants, thickening agents, gel formers, colourings, stabilisers, scents, pH regulators, osmoactive substances and other additives. The proportion of additives is preferably between 0.01% by weight and 30% by weight, in terms of the total weight of the composition.

It is particularly preferable for the composition according to the invention to contain medium-chain triglycerides (MCTs). Within the scope of the present invention, this means triglycerides or mixtures thereof, in which straight-chain fatty acids with chain lengths of C₈ to C₁₆ are esterified with glycerine. Triglycerides with fatty acids with chain lengths of C₈ to C₁₂ are particularly preferred. Products of this type are traded under various names (e.g. Myritol 312 and 318 or Miglylol 810 and 812).

The addition of salts, particularly those of sodium, potassium, magnesium, calcium, zinc and choline is also particularly favourable. Fluorides, chlorides, sulphates, phosphates, 2-aminoethylphosphates, glycolates, lactates, citrates, succinates, malates, fumarates, monomethyl fumarates, monoethyl fumarates, tartrates or mixtures thereof are preferred. The mixtures may, for example, be of natural origin (including sea salt of different provenance, e.g. from the Dead Sea). Chlorides, sulphates and fumarates of potassium, sodium and magnesium are particularly preferred.

The use of moisture retainers has also proved particularly successful within the scope of the present invention. This means physiologically compatible polyols, urea or mixtures thereof. Typical representatives are glycol, propylene glycol, butylene glycol, pentylene glycol, hexylene glycol, glycerine, inositol (inosit), sorbitol, (Sorbit), mannit, palatinite, maltodextrin, dextrin, cyclodextrin, glucose, fructose, lactose mannose, galactose and other saccharides. Glycerine, propylene glycol, pentylene glycol and urea are particularly preferred, propylene glycol being used in as low a dose as possible.

In accordance with the invention, polymer sulphonic acids are preferably used as gel formers and are practically partially neutralised. Ammonium acryloyldimethyltaurate-vinylpyrrolidone copolymers have proved particularly successful in this connection, particularly those with a Brookfield viscosity in accordance with DIN EN ISO 2555 in a range from 48,000 mPas to 65,000 mPas (measured as a 1% solution in deionised water at 20°C). The viscosity is preferably measured at at least one frequency in the range from 1 to 1000 Hz, particularly at 100 Hz. The proportion of sulphonic acid is preferably between 0.1% by weight and 2.5% by weight, particularly between 0.5% by weight and 1.5% by weight, in terms of the total weight of the composition.

A particularly preferred and widely-used embodiment of the solution according to the invention contains between
- 5% by weight and 90% by weight of water;
- 0.01% by weight and 5% by weight of plant extract b-1), particularly Dioscorea spec. extract, Pelvetia canaliculata extract and/or glycine soya extract;
- 0.01% by weight and 5% by weight of anti-ageing agent b-2), particularly Punica granatum extract and/or Phyllanthus emblica extract;
- 0.1% and 15% by weight of substance c), particularly at least one hydrated phospholipid;
- 0.1% by weight and 10% by weight of at least one gel former, in particular at least one polymer sulphonic acid;
- as well as other constituents, particularly the aforementioned constituents, in a concentration between 0% by weight and 60% by weight,
in terms of the total weight of the composition.

In this case the composition preferably contains 0.001% by weight to 1% by weight of hyaluronic acid.

In this connection, the figures for the plant extract refer to the respective dry weights following drying to constant weight at 105°C for at least 3 hours.

The composition according to the invention can fundamentally be made up in any formulation for percutaneous application, the composition preferably being formulated as a percutaneously-applicable gel having, for practical purposes, a viscosity between 1,000 mPas and 40,000 mPas, preferably between 12,000 and 25,000 mPas at 20°C, so that a gel formulated in this way can be distributed on the skin faultlessly and particularly smoothly. The viscosity is preferably measured at at least one frequency in the range from 1 to 1000 Hz, particularly at 100 Hz.

The composition preferably has a pH between 4.0 and 7.6, to ensure application of the composition according to the invention, particularly to test subjects with sensitive skin.

The composition according to the invention can be mixed using the usual mixers, known to a person skilled in the art, and subsequently homogenised with a jetstream mixer, rotor-stator mixer, high-pressure homogeniser, dissolver or other similar units.

Preferred areas of application of the composition according to the invention include its use to produce a cosmetic composition and to produce a pharmaceutical composition to change the hormone level in a human, particularly a woman, the composition then being applied percutaneously.

The composition according to the invention will be described in more detail below, using specimen embodiments, without thereby restricting the inventive concept.

### Example 1: Day cream for women from the age of 30 years

| | |
|---|---|
| Lamellar system (containing phospholipids) | 30.0% by weight |
| Punica granatum extract | 1.0% by weight |
| Dioscorea spec. extract | 0.5% by weight |
| Hydrocolloid | 1.2% by weight |
| Hyaluronic acid | 0.1% by weight |
| Glycerine | 5.0% by weight |
| Preservatives | 0.75% by weight |
| Perfume oils | 0.4% by weight |
| Oils | 15.0% by weight |
| UV protection | 3.0% by weight |
| Water | add 100% by weight |

### Example 2: Intensive eye concentrate for women from the age of 30 years

| | |
|---|---|
| Lamellar system (containing phospholipids) | 17.00% by weight |
| Punica granatum extract | 1.00% by weight |
| Dioscorea spec. extract | 0.20% by weight |
| Acmella oleaceae extract | 1.50% by weight |
| Liposomes | 1.50% by weight |
| Hydrocolloid | 1.20% by weight |
| Hyaluronic acid | 0.20% by weight |
| Glycerine | 3.00% by weight |
| Preservatives | 0.75% by weight |
| Perfume oil | 0.15% by weight |
| Oils | 8.50% by weight |
| UV protection | 3.00% by weight |
| Water | add 100% by weight |

### Example 3: Day cream for women from the age of 60 years

| | |
|---|---|
| Lamellar structures (containing phospholipids) | 45.00% by weight |
| Helvetica canaliculate extract | 1.00% by weight |
| Glycine soya extract | 0.10% by weight |
| Phyllantus emblica extract | 0.10% by weight |
| Hydrocolloid | 0.77% by weight |
| Hyaluronic acid | 0.05% by weight |
| Glycerine | 5.00% by weight |
| Preservatives | 0.75% by weight |
| Perfume oil | 0.50% by weight |
| Oils | 17.00% by weight |
| UV protection | 3.00% by weight |
| Water | add 100% by weight |

## Claims

1. A composition for percutaneous application, particularly for use on ageing and/or stressed skin, which contains
a) Water;
b) at least two percutaneously-applicable active substances and
c) at least one substance which forms a lamellar structure with water, and which contains at least one functional group of the general formula I
- CH₂ - N⁺ - (CH₃)₃, (Formula I).
**characterised in that** the composition
b-1) contains at least one plant extract containing at least one phytohormone, and
b)-2) at least one anti-ageing agent other than b-1.

2. The composition in accordance with claim 1, **characterised in that** the phytohormone affects the oestrogen, progesterone and/or testosterone level in a woman.

3. The composition in accordance with claim 2, **characterised in that** the composition contains at least one plant extract of Cimicifuga racemosa, Trifolium pratense, Angelica sinensis, Panax ginseng, Piper methysticum, Glycine max, Dioscorea spec., Helvetica spec., Humulus lupulus, Glycyrrhiza glabra, Urtica dioica, Sabal serrulata, Serenoa repens, Secale cereale, Avena sativa, Cucurbita pepo and/or Hypoxis rooperi.

4. The composition in accordance with at least one of the preceding claims, **characterised in that** the composition contains at least two different plant extracts.

5. The composition in accordance with claim 4, **characterised in that** the plant extracts affect at least two different hormone levels in the woman.

6. The composition in accordance with claim 5, **characterised in that** the composition contains Trifolium pratense extract : Dioscorea spec. extract in a ratio from 1:100 to 100:1, in terms of the dry weight of the extracts.

7. The composition in accordance with claim 5, **characterised in that** the composition contains glycine max extract : Pelvetica spec. extract in a ratio of 1:100 to 100:1, in terms of the dry weight of the extracts.

8. The composition in accordance with at least one of the preceding claims, **characterised in that** the composition contains Punica granatum extract, vitamin E, alpha-liponic acid, Phyllanthus emblica extract and/or Vitis vinifera extract.

9. The composition in accordance with at least one of the preceding claims, **characterised in that** the composition contains one or more extracts of apple (Malus domestica), pear (Pyrus communis), quince (Cydonia oblonga), morello cherry (Cerasus vulgaris), sweet cherry (Cerasus avium), plum (Prunus domestica), apricot (Armeniaca vulgaris), peach (Persica vulgaris), gooseberry (Ribes uva-crispa), blackcurrant (Ribes rubrum), raspberry (Rubus idaeus), blackberry (Rubus fruticosus), strawberry (Fragaria spec.), rosehip (Rosa spec.), pineapple (Ananas comosus), fig (Ficus carica), bilberry (Vaccinium myrtillus and other species of vaccinium), cranberry (Vaccinium vitis-idaea), elderberry (Sambucus nigra), cornelian cherry (Cornus mas), sallow thorn (Hippophae rhamnoides), sloe (Prunus spinosa), Oregon grape (Mahonia aquifolium), mango (Mangifera indica), Barbados cherry (Malpighia glabra), melon (Cucumis melo), water melon (Citrullus lanatus), pomegranate (Punica granatum), passion fruit (Passiflora edulis), durian fruit (Durio zibethinus), akee (Blighia sapida), lychee (Litchi chinensis), rambutan (Nephelium lappaceum), mangosteen (Garcinia mangostana), cashew apple (Anacardium occidentale), tamarind (Tamarindus indica), grape (Vitis vinifera), banana (Musa spec.), date (Phoenix dactylifera), papaya (Carica papaya), guava (Psidium guajava), Japanese persimmon (Diospyrus kaki), kiwi (Actinidia chinensis), physalis (Physalis peruviana), Cape gooseberry (Opuntia ficus-indica), star fruit (Averrhoa carambola), orange (Citrus sinensis), mandarin (Citrus deliciosa), grapefruit (Citrus paradisi), shaddock (Citrus maxima), lemon (Citrus limon), citron (Citrus medica), key lime (Citrus aurantiifolia), bitter orange (Citrus aurantium), kumquat (Fortunella margarita) and/or resveratrol.

10. The composition in accordance with at least one of the preceding claims, **characterised in that** the composition contains monoglycerides, diglycerides, preferably distilled medium-chain monoglycerides, sphingolipids, lecithin, phospholipids, fatty alcohols, fatty acids, soaps, mono- and/or diesters of fatty acids, sucrose, glucose and/or their derivatives, glucosidal, furanosidal and/or pyranosidal condensation products of fatty alcohols with glucose and/or sucrose and their polymer derivatives, mono- and/or diesters of glucosides with fatty acid derivatives, steroids, mono- and/or diesters of fatty acids and steroids and/or glycol derivatives from steroids as substance c).

11. The composition in accordance with at least one of the preceding claims, **characterised in that** the composition contains the anti-ageing agent b-1) in a quantity in a range from 0.001% by weight to 5% by weight in terms of the total weight of the composition.

12. The composition in accordance with at least one of the preceding claims, **characterised in that** the composition contains the anti-ageing agent b-2) in a quantity in a range from 0.001% by weight to 5% by weight in terms of the total weight of the composition.

13. The composition in accordance with at least one of the preceding claims, **characterised in that** the composition contains a hydrated phospholipid, and particularly a hydrated phosphatidylcholine, as substance c).

14. The composition in accordance with claim 13, **characterised in that** the hydrated phospholipid contains at least 60% by weight hydrated phosphatidylcholine.

15. The composition in accordance with at least one of the preceding claims, **characterised in that** the composition contains the substance c) in a concentration between 0.5% by weight and 30% by weight, preferably in a concentration between 1% by weight and 15% by weight in terms of the composition.

16. The composition in accordance with at least one of claims 13 to 15, **characterised in that** the hydrated phospholipid has a phase transition temperature over 30°C and below 70°C.

17. The composition in accordance with at least one of the preceding claims, **characterised in that** the compound c) is betaine, acetylcholine, choline, glycerophosphocholine, phosphatidylcholine, lysophosphatidylcholine, carnitine, acylcarnitine or sphingomyeline, mixtures and/or derivatives thereof.

18. The composition in accordance with at least one of the preceding claims, **characterised in that** the composition contains water in a concentration of between 5% by weight and 90% by weight, in terms of the total weight of the composition.

19. The composition in accordance with at least one of the preceding claims, **characterised in that** the composition also contains at least one preservative, one antioxidant, one thickening agent, one gel former one other additive and/or an alcohol, preferably a polyvalent alcohol.

20. The composition in accordance with at least one of the preceding claims, **characterised in that** the composition contains between
- 5% by weight and 90% by weight of water;
- 0.01% by weight and 5% by weight of plant extract b-1);
- 0.01% by weight and 5% by weight of anti-ageing agents b-2);
- 0.1% by weight and 15% by weight of substance c);
- 0.1% by weight and 10% by weight of a gel former;
- and other constituents in a concentration between 0% by weight and 60% by weight
in terms of the total weight of the dry weight of the extract or extracts.

21. The composition in accordance with at least one of the preceding claims, **characterised in that** the composition is formulated as a percutaneously-applicable gel and has a viscosity between 1,000 mPas and 40,000 mPas at 20°C.

22. The composition in accordance with at least one of the preceding claims, **characterised in that** the composition has a pH between 4.0 and 7.6.

23. The composition in accordance with at least one of the preceding claims, **characterised in that** the composition has the lamellar double membrane structure in a concentration between 15% and 99%, expressed in terms of the weight of the carrier substance contained in the composition.

24. The composition in accordance with at least one of the preceding claims, **characterised in that** the composition has a lamellar double membrane structure in which each individual double membrane is between 4 nm and 20 nm thick, preferably between 4 nm and 8 nm thick.

25. The use of a composition in accordance with at least one of the preceding claims to produce a cosmetic composition.

26. The use of a composition in accordance with at least one of claims 1 to 24 to produce a pharmaceutical composition to change the hormone level in a human.
